# EUROPEAN PATENT APPLICATION

(11) **EP 4 814 884 A1**
(43) Date of publication of application: **30.09.2026**
(21) Application number: 26160910.1
(22) Date of filing: 26.02.2026
(51) Int. Cl.: G06V 10/426, G06V 10/764, G06V 10/82, G06V 20/69, G16H 50/20, G16H 50/70, G16H 30/40, G16H 50/50

(54) **INFORMATION PROCESSING APPARATUS, INFORMATION PROCESSING METHOD, AND PROGRAM**

(30) Priority: 28.03.2025 JP 2025055493
(71) Applicant: Rakuten Group, Inc., Setagaya-ku Tokyo 158-0094 (JP)
(72) Inventor: SHARMA, Shreya, Tokyo, 158-0094 (JP); MAJUMDAR, Shantanu, Tokyo, 158-0094 (JP)
(74) Representative: Hoffmann Eitle

(57) **Abstract**

An information processing apparatus classifies cells in an image of a tumor microenvironment obtained from a patient into a plurality of cell groups, extracts positions, in the image, of the cells included in the plurality of cell groups, creates a graph by assigning nodes to the cells included in the plurality of cell groups based on the extracted positions, connecting the nodes with edges, assigning to each of the nodes a feature quantity indicating a feature of the corresponding cell, and assigning to each of the edges a feature quantity indicating a relationship between two cells corresponding to two nodes connected by the edge, and models, using a graph neural network (GNN), spatial relationships between cells in the graph based on the assigned feature quantities of the nodes and the edges.

## Description

### CROSS-REFERENCE TO RELATED APPLICATION(S)

This application claims priority to Japanese patent application No. 2025-055493, filed on March 28, 2025; the entire contents of which are incorporated herein by reference.

### TECHNICAL FIELD

The present invention relates to technology for analysis in a tumor microenvironment using a graph neural network.

### BACKGROUND ART

Analyzing spatial interactions between tumor cells and immune cells in a tumor microenvironment (TME) is advantageous due to providing predictive and prognostic value for therapeutic efficacy. For example, Non-Patent Document 1 discloses that spatial interactions between tumor cells and immune cells are analyzed by quantifying the spatial interactions between adjacent cells using distance metrics. For example, interactions between tumor cells and immune cells closest to the tumor cells have been quantified.

Non-Patent Document 1: James Monkman et al. "Spatial insights into immunotherapy response in non-small cell lung cancer (NSCLC) by multiplexed tissue imaging", Journal of Translational Medicine, https://doi.org/10.1186/s12967-024-05035-8

### SUMMARY OF THE INVENTION

With the proximity-based method disclosed in Non-Patent Document 1, interactions with tumor cells are quantified only for the immune cells closest to the tumor cells, and therefore information is lacking on the cell positions of other immune cells and interactions they have with the tumor cells. Therefore, it has not been possible to sufficiently analyze the spatial interactions between multiple types of cells, such as tumor cells and immune cells, in a tumor microenvironment.

In view of the foregoing problem, the present disclosure provides a new technique for analyzing spatial interactions between multiple types of cells in a tumor microenvironment.

In order to solve the foregoing problem, an information processing apparatus according to one aspect of the present invention includes: a classification unit configured to classify cells in an image of a tumor microenvironment obtained from a patient into a plurality of cell groups; a spatial information extraction unit configured to extract positions, in the image, of the cells included in the plurality of cell groups; a graph creation unit configured to create a graph by assigning nodes to the cells included in the plurality of cell groups based on the extracted positions, connecting the nodes with edges, assigning to each of the nodes a feature quantity indicating a feature of the corresponding cell, and assigning to each of the edges a feature quantity indicating a relationship between two cells corresponding to two nodes connected by the edge; and a graph modeling unit configured to, using a graph neural network (GNN), model spatial relationships between cells in the graph based on the assigned feature quantities of the nodes and the edges.

In order to solve the foregoing problem, an information processing method according to one aspect of the present invention includes: classifying cells in an image of a tumor microenvironment obtained from a patient into a plurality of cell groups; extracting positions, in the image, of the cells included in the plurality of cell groups; creating a graph by assigning nodes to the cells included in the plurality of cell groups based on the extracted positions, connecting the nodes with edges, assigning to each of the nodes a feature quantity indicating a feature of the corresponding cell, and assigning to each of the edges a feature quantity indicating a relationship between two cells corresponding to two nodes connected by the edge; and modeling, using a graph neural network (GNN), spatial relationships between cells in the graph based on the assigned feature quantities of the nodes and the edges.

In order to solve the foregoing problem, a program according to one aspect of the present invention is a program for causing a computer to execute information processing, the program causing the computer to execute: classification processing of classifying cells in an image of a tumor microenvironment obtained from a patient into a plurality of cell groups; spatial information extraction processing of extracting positions, in the image, of the cells included in the plurality of cell groups; graph creation processing of creating a graph by assigning nodes to the cells included in the plurality of cell groups based on the extracted positions, connecting the nodes with edges, assigning to each of the nodes a feature quantity indicating a feature of the corresponding cell, and assigning to each of the edges a feature quantity indicating a relationship between two cells corresponding to two nodes connected by the edge; and modeling processing of modeling, using a graph neural network (GNN), spatial relationships between cells in the graph based on the assigned feature quantities of the nodes and the edges.

According to the present invention, it is possible to analyze spatial interactions between multiple types of cells in a tumor microenvironment using information on cell positions and interactions.

A person skilled in the art will be able to understand the above-stated object, aspect, and advantages of the present invention, as well as other objects, aspects, and advantages of the present invention that are not mentioned above, from the following modes for carrying out the invention by referring to the accompanying drawings and claims.

### BRIEF DESCRIPTION OF THE DRAWINGS

FIG. 1 shows an example of the functional configuration of an information processing apparatus according to an embodiment.
FIG. 2 shows a flowchart of overall processing in a first embodiment.
FIG. 3 shows an example of images corresponding to one or more processing steps in the first embodiment.
FIG. 4 shows a conceptual diagram of a procedure for training a responder prediction model using subgraphs in the first embodiment.
FIG. 5 shows a conceptual diagram of a prediction procedure that uses the responder prediction model in the first embodiment.
FIG. 6 shows a flowchart of overall processing in a second embodiment.
FIG. 7 shows an example of images corresponding to one or more processing steps in the second embodiment.
FIG. 8 shows a conceptual diagram of a procedure for training a responder prediction model using subgraphs in the second embodiment.
FIG. 9 shows a conceptual diagram of a prediction procedure that uses the responder prediction model in the second embodiment.
FIG. 10 shows an example of the hardware configuration of an information processing apparatus according to an embodiment.

### EMBODIMENTS OF THE INVENTION

Embodiments of the present invention will now be described in detail with reference to the accompanying drawings. Out of the component elements described below, elements with the same functions have been assigned the same reference numerals, and description thereof is omitted. Note that the embodiments disclosed below are mere example implementations of the present invention, and it is possible to make changes and modifications as appropriate according to the configuration and/or various conditions of the apparatus to which the present invention is to be applied. Accordingly, the present invention is not limited to the embodiments described below. The combination of features described in these embodiments may include features that are not essential when implementing the present invention.

In the present disclosure, in an image of a tumor microenvironment (TME) that includes tumor cells obtained from a patient (subject), cells are classified into cell groups, spatial information of the various types of cells is extracted (specifically, cell positions are extracted (identified)), and a graph having nodes corresponding to the cells and edges corresponding to connections between the cells is created based on the spatial information. Here, the patient is either a responder or a non-responder. However, these classes are only one example of a predictive use case, and other classes such as short/long survival (prognostic use case) and cancerous/non-cancerous (detective use case) are also possible. Then, the created graph is modeled based on node features (i.e., cell features) and edge features (i.e., inter-cell features), and a graph for a graph neural network (GNN) is modeled. The positions of the nodes correspond to the positions of the cells, and the edges correspond to interactions between the nodes. Accordingly, spatial interactions between multiple types of cells in a TME are analyzed using information on cell position and interactions. Next, information that is based on the modeled graph is associated with a label indicating "responder" or "non-responder", which is a true label (ground truth data), and a responder prediction model is trained. In the present disclosure, graph-based information includes, but is not limited to, multiple subgraphs extracted from a modeled graph. The responder prediction model is a GNN-based machine learning model for learning the relationship between a subgraph extracted from the modeled graph and the true label. In other words, the responder prediction model is trained to learn the relationship between a subgraph and a responder or a non-responder. Then, multiple subgraphs obtained from an image of a tumor microenvironment of a target patient are input to the trained responder prediction model, and it is predicted whether the target patient is a responder or non-responder.

A TME is microtissue that includes tumor cells (tumor tissue) and a surrounding mixture of blood vessels, stromal cells, immune cells that infiltrate the tumor cells, and normal tissue. This can also be described as a network constructed between multiple types of cells, including tumor cells and immune cells. This is known to have many effects on tumor initiation, development, and progression. Conventional cancer treatments have targeted cancer cells, but in recent years, the TME has attracted attention as a new target.

In the present disclosure, the terms "responder" and "non-responder" correspond to a responder or a non-responder with respect to a given treatment. For example, the term "responder" may correspond to a patient who has a relatively high level of immune cell infiltration into tumor cells upon receiving a treatment, while the term "non-responder" may correspond to a patient who has a relatively low level of immune cell infiltration into tumor cells upon receiving a treatment. Additionally, the term "responder" may correspond to a patient who shows a certain response to a cancer immunotherapy, while the term "non-responder" may correspond to a patient who does not show a certain response to a cancer immunotherapy.

A GNN is a type of deep learning model designed to process data having a graph structure. In the present disclosure, a GNN is constructed by assigning a node to each tumor cell and immune cell and connecting the nodes with edges (links). The constructed GNN is trained using features of the nodes, features of the edges between nodes, and a true label.

### Functional Configuration of Information processing apparatus

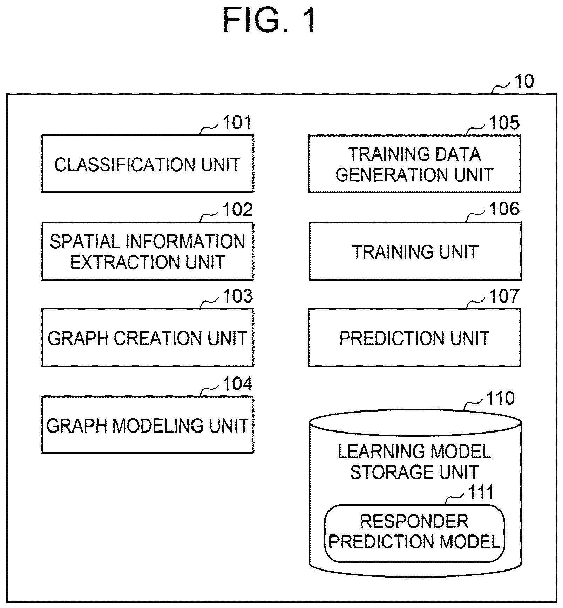

FIG. 1 shows an example of the functional configuration of an information processing apparatus 10 configured to be able to implement the processing described in the present disclosure. As shown in FIG. 1, the information processing apparatus 10 has a classification unit 101, a spatial information extraction unit 102, a graph creation unit 103, a graph modeling unit 104, a training data generation unit 105, a training unit 106, a prediction unit 107, and a learning model storage unit 110. The learning model storage unit 110 is configured to store a responder prediction model 111. For example, the learning model storage unit 110 is configured to store the architecture and various parameters of the responder prediction model 111.

An overview of processing performed by the functional components will be described below. The classification unit 101 classifies cells in an image of a tumor microenvironment obtained from a patient into cell groups. There may be two or more cell groups, and the number of cell groups depends on the use case. The method proposed here enables studying not only interactions between tumor cells and immune cells, but also interactions between immune cells, interactions between tumor cells, and interactions between tumor cells and other types of cells. The spatial information extraction unit 102 extracts position information on the positions where cells included in a cell group are located in an image. The graph creation unit 103 creates a graph by assigning a node to each cell included in the cell group based on the extracted position information and then connecting the nodes with edges. Each node is assigned a feature quantity that indicates a cell feature, and each edge is assigned a feature quantity that indicates a relationship between the two cells that correspond to the two nodes connected by the edge. The graph modeling unit 104 uses a graph neural network (GNN) to model or learn the spatial relationships between cells in the graph created by the graph creation unit 103, based on the assigned feature quantities of the nodes and edges. In the present disclosure, the processing of modeling or learning may be referred to simply as modeling.

Hereinafter, two embodiments, namely a first embodiment and a second embodiment, will be specifically described with reference to the functional configuration of the information processing apparatus 10 shown in FIG. 1.

### First Embodiment

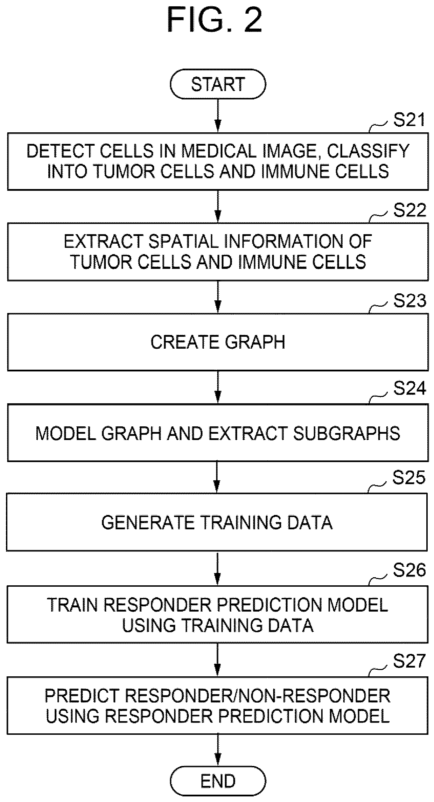

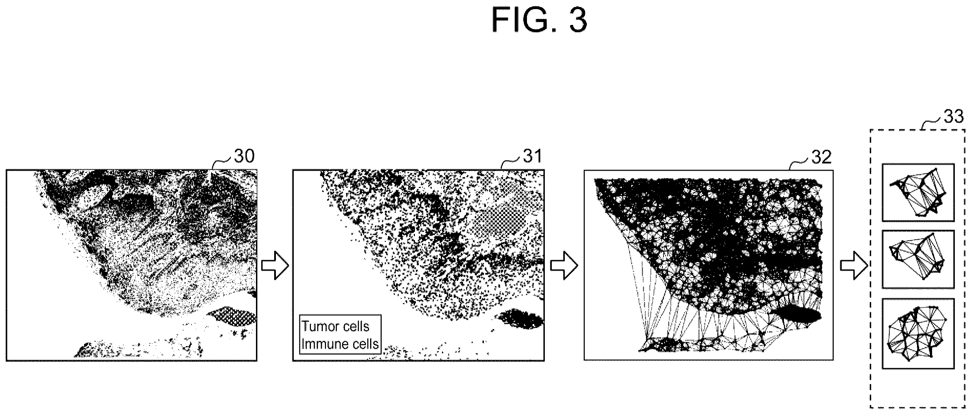

FIG. 2 shows a flowchart of overall processing according to the present embodiment. Also, FIG. 3 shows an example of images corresponding to one or more processing steps shown in FIG. 2. First, the classification unit 101 detects cells in an image (hereinafter referred to as a medical image) of a tumor microenvironment that includes tumor cells obtained from a patient (responder or non-responder), and classifies the detected cells into tumor cells and immune cells (S21). In the present embodiment, the classification unit 101 classifies the detected cells into two types of cell groups, namely tumor cells and immune cells, but is not limited to this, and may classify cells into three or more types of cell groups, such as tumor cells, immune cells, and stromal cells. This enables analyzing not only interactions between tumor cells and immune cells, but also interactions between immune cells, interactions between tumor cells, and interactions between tumor cells and other types of cells. In the present embodiment, the medical image is an image in which multiple types of cell groups can be identified (including an image in which they can be visualized), such as a multiplex immunofluorescence (mIF) image. An mIF image is an image in which different types of cells or the like have been stained with multiple fluorescent dye-labeled antibodies. An image 30 in FIG. 3, which is an example of an mIF image, shows a tumor microenvironment that includes tumor cells, and in this image, cells are identified by multiple types of antibodies (note that although this image 30 is a black and white image, the actual mIF image is a multi-color image).

Cell detection in a medical image can be performed as described below, for example. In the case where multiple types of cells in a medical image have been identified by different colors, cells may be detected by identifying the various colors. Furthermore, in the case where multiple types of cells in a medical image have been identified by one or more pixels corresponding to a predetermined numerical value, cells may be detected using a predetermined threshold value. Also, watershed segmentation may be used to detect cells. Deep learning may also be used to detect cells. In the case of using deep learning, cells can be detected by, for example, using an encoder-decoder based architecture or bounding box detection.

After cells are detected, the cells can be classified into multiple types of cell groups using, for example, predetermined rules that are based on cell structure, or predetermined features (e.g., cell shape, size, texture, intensity, etc.). Additionally, types of cell groups may be classified using machine learning/deep learning. In the case of using machine learning/deep learning, cells can be classified by using a classifier such as random forest, logistic regression, support vector machine, convolutional neural network, or visual transformer. An image 3 1 in FIG. 3 is an example of an image in which cells that were detected in the image 30 have been classified into tumor cells and immune cells.

After cells are detected in the medical image and classified into tumor cells and immune cells, the spatial information extraction unit 102 extracts spatial information of the tumor cells and the immune cells (S22). In the present embodiment, the spatial information extraction unit 102 defines a predetermined two-dimensional coordinate system (i.e., a two-dimensional XY coordinate system) in the medical image, and extracts (identifies) the cell positions of tumor cells and immune cells in the two-dimensional coordinate system. The spatial information extraction unit 102 extracts spatial information regarding the tumor cells and the immune cells by extracting the X coordinate and Y coordinate of each of the tumor cells and the immune cells in the image subjected to classification into tumor cells and immune cells.

After the spatial information regarding the tumor cells and the immune cells is extracted, the graph creation unit 103 creates a graph based on the spatial information (S23). In the present embodiment, the graph creation unit 103 creates a graph by assigning nodes at the positions of the tumor cells and the immune cells specified by X coordinates and Y coordinates, and then connecting the nodes with edges. Here, each node may be connected to the nearest adjacent node by an edge. Furthermore, each node may be connected to a node located within a predetermined range by an edge. Furthermore, each node may be connected to a predetermined number of adjacent nodes by edges. Furthermore, nodes may be connected by edges using Delaunay triangulation. Delaunay triangulation is a technique of creating a contiguous, non-overlapping triangle mesh from a set of points (i.e., nodes). Alternatively, a hierarchical technique may be used to connect nodes with edges. For example, a predetermined direction is defined in a two-dimensional coordinate system (XY coordinate system), and an edge is used to connect a node higher in the predetermined direction to one or more nodes lower in the predetermined direction. Furthermore, the K-nearest neighbors technique may be used to connect nodes with edges. This creation processing obtains a graph having nodes corresponding to the tumor cells and the immune cells, and edges connecting the nodes.

Furthermore, the graph creation unit 103 assigns to each node in the graph a feature quantity indicating a feature of the corresponding cell (i.e., a feature quantity of the cell corresponding to the node), and assigns to each edge a feature quantity indicating a relationship between two cells corresponding to the two connected nodes (i.e., a feature quantity that pertains to the two cells corresponding to the two nodes connected by the edge, and is a feature quantity of an interaction between edges). The feature quantity indicating the feature of the corresponding cell may include, for example, at least any one of the type of the cell (e.g., a feature related to the function of the cell), the size of the cell, a feature of a biomarker expression level in the cell, a relationship with a node corresponding to an adjacent cell (e.g., the presence or absence of the same type of cell in the vicinity), and the center coordinates of the node corresponding to the cell. The feature quantity indicating the relationship between two cells may include, for example, at least any one of the type of edge (the type of connection between the cells (e.g., information indicating whether the connection is between cells of the same type or between cells of different types)) and an edge distance (e.g., the distance between the nodes corresponding to the two cells). An image 32 in FIG. 3 is an example of a graph in which the nodes corresponding to the tumor cells and the immune cells in the image 31 are connected by edges between nodes.

Next, the graph modeling unit 104 uses the GNN to model the spatial relationships between cells in the graph created by the graph creation unit 103, based on the assigned feature quantities of the nodes and edges (S24). The processing performed by the graph modeling unit 104 may include message passing, that is to say exchanging node features between nodes, and aggregating neighborhood information around the nodes and exchanging information between nodes. Message passing enables pairs of nodes to learn more about interactions between nodes. Examples of a GNN (i.e., a neural network having a GNN structure) include GCN (Graph Convolutional Network), GIN (Graph Isomorphism Network), GRAPHSAGE (Graph Sample and Aggregation), and GAT (Graph Attention Network). These GNN examples represent different methods of aggregating neighborhood information and propagating messages between nodes. By using such a graph modeling procedure, spatial interactions between multiple types of cells are analyzed (learned) using information on cell positions and interactions.

Furthermore, the graph modeling unit 104 extracts multiple subgraphs from the modeled graph, as graph-based information (S24). The graph modeling unit 104 extracts a subgraph from the modeled graph on the condition that the subgraph has at least (N+1) nodes (N is an integer greater than or equal to 2) and has N hops (N edges continuous with each other). This provision of the condition of having N hops means extracting a subgraph that better reflects the relationships between nodes due to edge connections. Since the extracted subgraphs are used as training data for training the responder prediction model 111, it is preferable that the graph modeling unit 104 extracts subgraphs from the entire modeled graph as much as possible, rather than extracting subgraphs locally from the modeled graph. This is because subgraphs with various patterns are extracted from the entire modeled graph, which contributes to accurate learning. A subgraph group 33 in FIG. 3 is an example of extracted subgraphs, and although the subgraph group 33 includes three subgraphs, it may include more than three subgraphs.

Next, the training data generation unit 105 generates training data for the responder prediction model 111 (S25). The training data is made up of the subgraphs extracted in S24 and a label indicating "responder" or "non-responder" as a true label. The label indicating "responder" or "non-responder" is determined based on whether the patient corresponding to the original medical image from which the subgraphs were extracted is a responder or a non-responder. As described above, each of the subgraphs has at least (N+1) nodes (N is an integer greater than or equal to 2) and N hops (N edges continuous with each other).

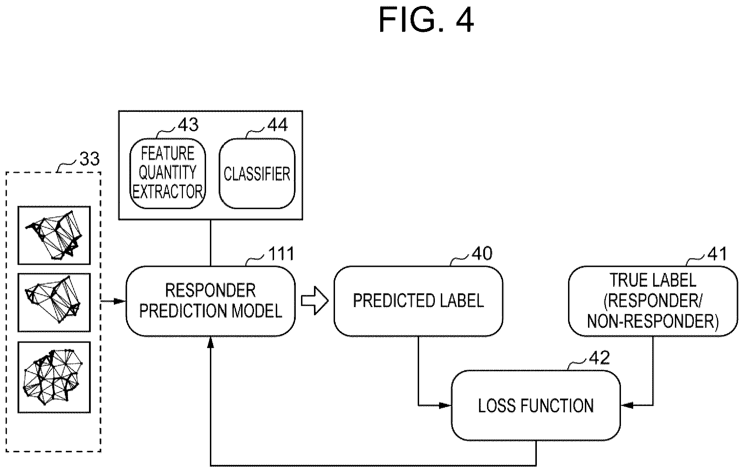

After the training data is generated, the training unit 106 uses the training data to train the responder prediction model 111 (S26). The responder prediction model 111 is a GNN for learning the relationship between subgraphs and a true label (a label indicating "responder or "non-responder"). FIG. 4 shows a conceptual diagram of a procedure for training the responder prediction model 111 using subgraphs. In FIG. 4, each subgraph in the subgraph group 33 is associated with a true label 41, and the training unit 106 inputs each subgraph in the subgraph group 33 into the responder prediction model 111 and obtains an output (i.e., a predicted label 40). The responder prediction model 111 according to the present embodiment includes, for example, a feature quantity extractor 43 and a classifier 44. The feature quantity extractor 43 extracts, from the input subgraph, a feature vector indicating a feature quantity of the subgraph, and the classifier 44 classifies the feature vector to predict a label indicating "responder" or "non-responder" and output the predicted label 40.

After the predicted label 40 is output, the training unit 106 trains the responder prediction model 111 using a loss function 42, which is a function that derives the magnitude of the difference (loss) between the true label 41 (i.e., the correct label) associated with the input subgraph and the predicted label. Specifically, the training unit 106 trains the responder prediction model 111 so as to reduce the value of the loss derived from the loss function 42. The trained responder prediction model 111 is configured to, upon receiving input of a subgraph, predict and output a label indicating "responder" or "non-responder" for the input subgraph. Specifically, the trained responder prediction model 111 is configured to predict and output information indicating whether the original medical image corresponding to the graph that corresponds to the input subgraph was obtained from a responder patient or a non-responder patient.

In this way, in the present embodiment, the responder prediction model 111 is trained using subgraphs in which each node has a node feature and each edge has an edge feature. Accordingly, the responder prediction model 111 can learn the relationship between a subgraph and a responder or a non-responder based on features of cells and features between cells.

After the responder prediction model 111 is trained, the prediction unit 107 uses the trained responder prediction model 111 to predict whether a target patient is a responder or a non-responder based on a medical image that includes a tumor microenvironment obtained from the target patient (S27). Specifically, the information processing apparatus 10 performs the processing of S21 to S24 on the medical image obtained from the target patient to extract multiple subgraphs. Then, the prediction unit 107 inputs each of the subgraphs extracted for the target patient to the trained responder prediction model 111, and predicts a label indicating "responder" or "non-responder" for the input subgraph. The prediction unit 107 predicts a label indicating "responder" or "non-responder" for all of the subgraphs extracted for the target patient, and thus obtains multiple predicted labels. Thereafter, the prediction unit 107 aggregates the predicted labels to ultimately predict (determine) whether the target patient is a responder or a non-responder. For example, the prediction unit 107 aggregates the predicted labels to ultimately determine a responder prediction score that indicates the likelihood that the target patient is a responder. This score is expressed by, for example, a numerical value between 0 and 1, where the closer the score is to 1, the higher the likelihood is that the target patient is a responder.

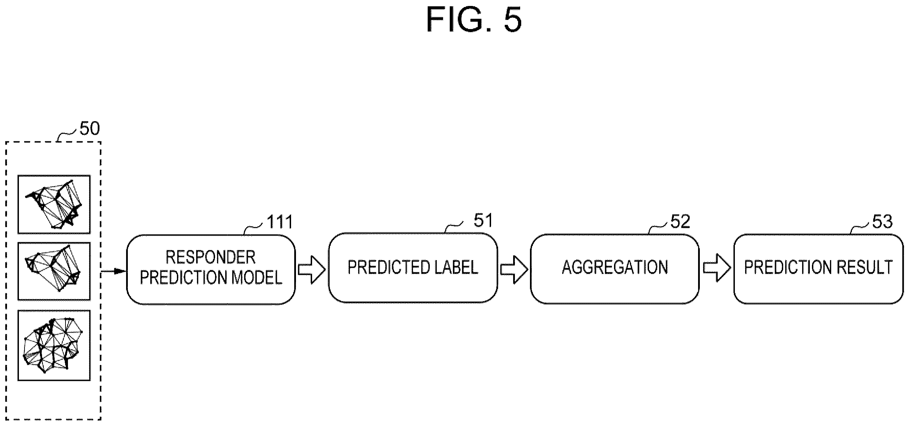

FIG. 5 shows a conceptual diagram of a prediction procedure that uses the responder prediction model 111. In FIG. 5, a subgraph group 50 includes multiple subgraphs extracted by performing the processing of S21 to S24 on a medical image that includes a tumor microenvironment obtained from a target patient. The prediction unit 107 inputs each of the subgraphs included in the subgraph group 50 to the trained responder prediction model 111, predicts a label indicating "responder" or "non-responder" for the input subgraph, and obtains a predicted label 51. The prediction unit 107 predicts a label indicating "responder" or "non-responder" for all of the subgraphs included in the subgraph group 50, thereby obtaining multiple predicted labels 51. Thereafter, the prediction unit 107 aggregates the predicted labels 51 (aggregation 52) to ultimately obtain a prediction result 53 indicating whether the target patient is a responder or a non-responder.

The aggregation 52 of the predicted labels 51 indicating "responder" or "non-responder" is statistical aggregation, and includes, for example, taking a majority vote of the predicted labels 51. In other words, the prediction unit 107 may obtain, as a final prediction result 53, whichever label is indicated the most among the predicted labels 51. Alternatively, the prediction unit 107 may assign "1" to the label indicating "responder" and "0" to the label indicating "non-responder" among the predicted labels 51, and obtain the average value of the total values of the labels as the final prediction result 53. In this case, a value between 0 and 1 is displayed as the prediction result 53 indicating the likelihood of being a responder.

In this way, the information processing apparatus 10 according to the first embodiment obtains spatial information (i.e., cell positions) regarding cells that were detected in a medical image and classified, assigns nodes to cells based on the spatial information, connects the nodes with edges, and models a graph using features of the nodes and features of the edges (i.e., interaction information). Accordingly, spatial interactions between multiple types of cells in a tumor microenvironment can be analyzed using information on cell positions and interactions. Then, the information processing apparatus 10 trains the responder prediction model 111 using subgraphs extracted from the modeled graph and a true label indicating "responder" or "non-responder". Accordingly, the responder prediction model 111 learns a relationship between a subgraph and a responder or a non-responder. The responder prediction model 111 can learn the relationship between a subgraph and a responder or a non-responder based on cell features and features between cells. Also, the information processing apparatus 10 uses the trained responder prediction model 111 to predict whether a patient is a responder or a non-responder based on subgraphs extracted from a medical image obtained from the target patient, and therefore, high prediction accuracy is expected for such prediction.

### Second Embodiment

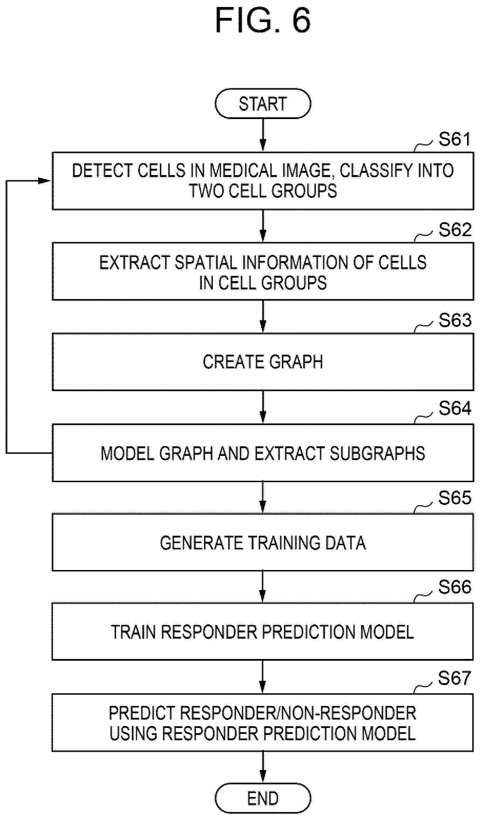

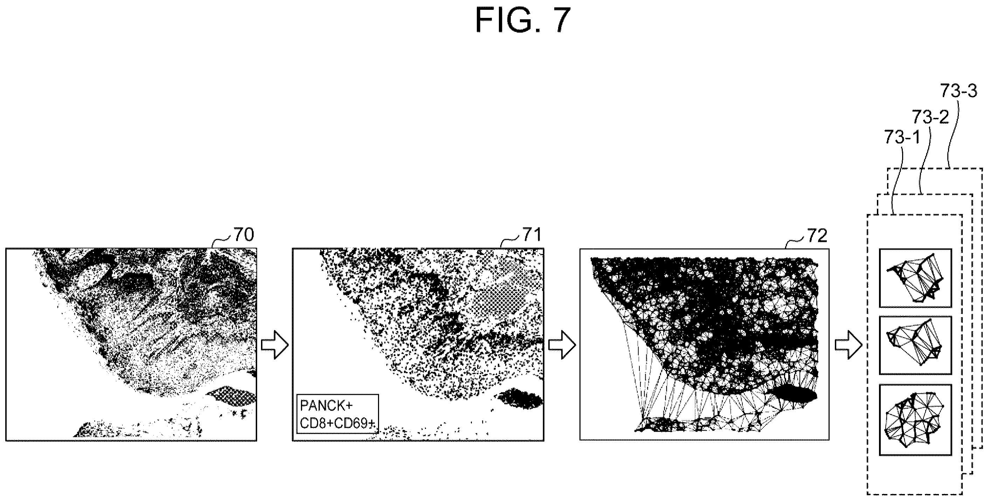

Next, a second embodiment will be described. Note that descriptions will be omitted for matter the same as in the first embodiment. FIG. 6 shows a flowchart of overall processing according to the present embodiment. FIG. 7 shows an example of images corresponding to one or more processing steps shown in FIG. 6. First, the classification unit 101 detects cells in an image (medical image) of a tumor microenvironment including tumor cells obtained from a patient (responder or non-responder), and classifies the detected cells into two types of cell groups (a first cell type group and a second cell type group) based on the phenotype (phenotype marker) of the cells (S61). The first cell type group includes cells of a first phenotype, the second cell type group includes cells of a second phenotype, and the term "cell type pair" will be used to refer to a pair of cells including a cell of the first phenotype and a cell of the second phenotype. In the present embodiment, the classification unit 101 ultimately classifies each of various cell type pairs into two types of cell groups, and here, classifies the first cell type pair into two types of cell groups. In the present embodiment, the medical image is an image in which multiple types of cells can be identified (including an image in which cells can be visualized), such as an mIF image. An image 70 in FIG. 7, which is an example of an mIF image, shows a tumor microenvironment that includes tumor cells, and in this image, cells are identified by multiple types of antibodies (note that although this image 70 is a black and white image, the actual mIF image is a multi-color image).

Cell detection in a medical image can be performed by a procedure similar to the procedure of S21 in FIG. 2 described in the first embodiment. After cells are detected, if the image 70 is an mIF image, classification into the two types of cell groups can be performed using the staining color. For example, a cell type pair may include PANCK+ cells and CD8+ and CD69+ cells. In this case, based on the image 70, the cell type pair can be classified into a first cell group including the PANCK+ cells and a second cell group including the CD8+ and CD69+ cells. PANCK is an abbreviation for Pan-Cytokeratin. An image 71 in FIG. 7 is an example of an image classified into two types of cell groups based on the image 70.

After cells are detected in the medical image and classified into two types of cell groups, the spatial information extraction unit 102 extracts spatial information of the cells included in each of the two types of cell groups (S62). In the present embodiment, the spatial information extraction unit 102 defines a predetermined two-dimensional coordinate system (i.e., a two-dimensional XY coordinate system) in the medical image, and extracts (identifies) the positions of the cells in the first cell group and the second cell group in the two-dimensional coordinate system. The spatial information extraction unit 102 extracts spatial information of the cells included in the first cell group and the second cell group by extracting the X coordinates and Y coordinates of the cells included in the first cell group and the second cell group in the image subjected to classification into the two types of cell groups.

After the spatial information regarding the cells in the first cell group and the second cell group is extracted, the graph creation unit 103 creates a graph based on the spatial information (S63). In the present embodiment, the graph creation unit 103 creates a graph by assigning nodes at the positions of the cells in the first cell group and the second cell group specified by X coordinates and Y coordinates, and then connecting the nodes with edges. Furthermore, the graph creation unit 103 assigns to each node in the graph a feature quantity indicating a feature of the cell (i.e., a feature of the cell corresponding to the node), and assigns to each edge a feature quantity indicating a relationship between two cells corresponding to the two connected nodes (i.e., a feature that pertains to the two cells corresponding to the two nodes connected by the edge, and is a feature of an interaction between edges). The procedure for creating the graph is similar to the procedure in S23 of FIG. 2 described in the first embodiment. An image 72 in FIG. 7 is an example of a graph in which nodes corresponding to the cells in the first cell group and the second cell group in image 71 are connected by edges between nodes.

Next, the graph modeling unit 104 uses a GNN to model the spatial relationships between cells in the graph created by the graph creation unit 103, based on the assigned feature quantities of the nodes and edges (S64). Furthermore, the graph modeling unit 104 extracts multiple subgraphs from the modeled graph (S64). The procedure for graph modeling and subgraph extraction is similar to the procedure in S24 of FIG. 2 described in the first embodiment. A subgraph group 73-1 in FIG. 7 is an example of extracted subgraphs, and although the subgraph group 73-1 includes three subgraphs, it may include more than three subgraphs.

After the processing up to this point is completed, the procedure returns to S61, and the classification unit 101 classifies the cells detected in the medical image into two types of cell groups (first cell group and second cell group) based on the cell phenotype (S61). Since the first cell type pair has already been classified into two types of cell groups, in the second execution of the processing of S61, the classification unit 101 classifies the second cell type pair, which is different from the first cell type pair, into two types of cell groups. Then, the spatial information extraction unit 102 extracts spatial information of the cells in the two types of cell groups (S62), and the graph creation unit 103 creates a graph based on the spatial information (S63). Thereafter, the graph modeling unit 104 performs graph modeling, constructs a graph for the GNN, and extracts multiple subgraphs from the constructed graph (S64). Accordingly, by repeating the processing of S61 to S64 multiple times (two or more times), subgraph groups are generated for two types of cell groups for each of multiple cell type pairs. Note that the processing repetitions may be performed in parallel, and are not limited to a specific order for the multiple cell type pairs. Subgraph groups 73-1 to 73-3 in FIG. 7 show subgraphs generated by performing the processing from S61 to S64 three times. The subgraph group 73-1 includes subgraphs generated for the first cell type pair. Similarly, the subgraph group 73-2 includes subgraphs generated for the second cell type pair, and the subgraph group 73-3 includes subgraphs generated for the third cell type pair.

Next, the training data generation unit 105 generates training data for the responder prediction model 111 (S65). The training data is made up of multiple subgraphs and a label indicating "responder" or "non-responder" as a true label. In the present embodiment, the training data generation unit 105 generates training data that includes multiple subgraphs included in subgraph groups respectively generated for multiple cell type pairs, and true labels.

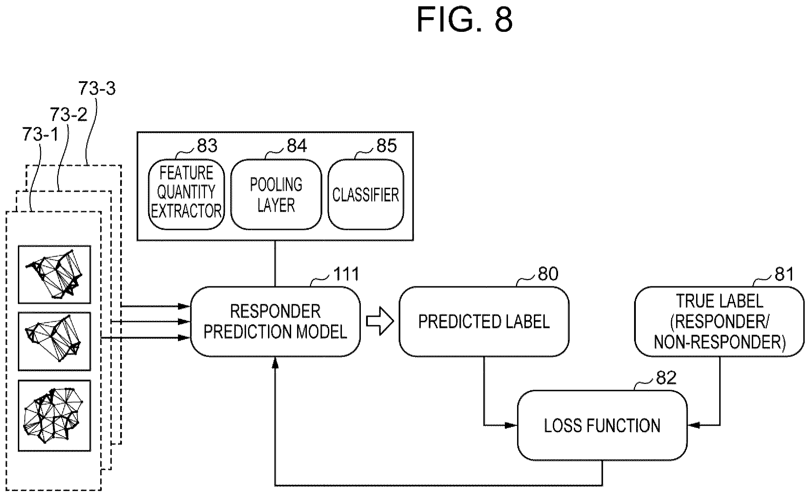

After the training data is generated, the training unit 106 uses the training data to train the responder prediction model 111 (S66). The responder prediction model 111 is a GNN for learning the relationship between subgraphs and a true label (a label indicating "responder or "non-responder"). FIG. 8 shows a conceptual diagram of a procedure for training the responder prediction model 111 using subgraphs. In FIG. 8, the subgraphs included in the subgraph groups 73-1 to 73-3 are associated with a true label 81, and the training unit 106 inputs the subgraphs of each of subgraph groups 73-1 to 73-3 into the responder prediction model 111 and obtains an output (i.e., a predicted label 80).

The responder prediction model 111 according to the present embodiment includes, for example, a feature quantity extractor 83, a pooling layer 84, and a classifier 85. The feature quantity extractor 83 extracts, from the input subgraph, a feature vector representing a feature quantity of the subgraph. Since the subgraph groups 73-1 to 73-3 each have a different cell type pair, the feature quantity extractor 83 extracts feature vectors in a different space (feature vector space) for each of the subgraph groups 73-1 to 73-3. The pooling layer 84 reduces the size of feature vectors extracted in different spaces. The classifier 85 is configured to classify the size-reduced feature vectors and output a predicted label 80.

After the predicted label 80 is output, the training unit 106 trains the responder prediction model 111 using a loss function 82, which is a function that derives the magnitude of the difference (loss) between the true label 81 (i.e., the correct label) associated with the input subgraph and the predicted label 80. Specifically, the training unit 106 trains the responder prediction model 111 so as to reduce the value of the loss derived from the loss function 82. The trained responder prediction model 111 is configured to, upon receiving input of a subgraph, predict and output a label indicating "responder" or "non-responder" for the input subgraph. Specifically, the trained responder prediction model 111 is configured to predict and output information indicating whether the original medical image corresponding to the graph that corresponds to the input subgraph was obtained from a responder patient or a non-responder patient.

After the responder prediction model 111 is trained, the prediction unit 107 uses the trained responder prediction model 111 to predict whether a target patient is a responder or a non-responder based on a medical image that includes a tumor microenvironment obtained from the target patient (S67). Specifically, the information processing apparatus 10 performs the processing of S61 to S64 multiple times on the medical image obtained from the target patient to extract multiple subgraphs for multiple cell type pairs. Then, the prediction unit 107 inputs each of the subgraphs extracted for the target patient to the trained responder prediction model 111, and predicts a label indicating "responder" or "non-responder" for the input subgraph. The prediction unit 107 predicts a label indicating "responder" or "non-responder" for all of the subgraphs extracted for the target patient, and thus obtains multiple predicted labels. Thereafter, the prediction unit 107 aggregates the predicted labels to ultimately predict (determine) whether the target patient is a responder or a non-responder. For example, the prediction unit 107 aggregates the predicted labels to ultimately determine a responder prediction score that indicates the likelihood that the target patient is a responder. This score is expressed by, for example, a numerical value between 0 and 1, where the closer the score is to 1, the higher the likelihood is that the target patient is a responder.

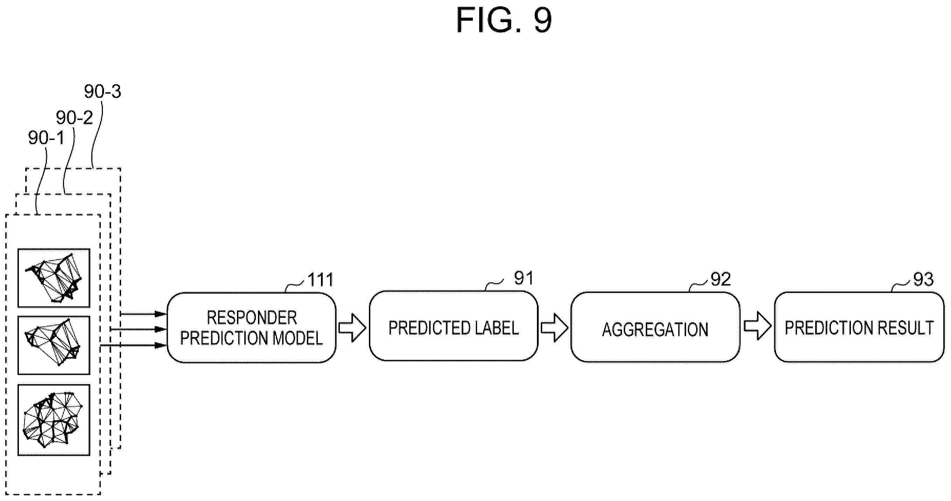

FIG. 9 shows a conceptual diagram of a prediction procedure that uses the responder prediction model 111. In FIG. 9, subgraph groups 90-1 to 90-3 include subgraphs extracted by performing the processing of S61 to S64 three times on a medical image containing a tumor microenvironment obtained from a target patient. In other words, the subgraph group 90-1 includes multiple subgraphs generated for a first cell type pair, the subgraph group 90-2 includes multiple subgraphs generated for a second cell type pair, and the subgraph group 90-3 includes multiple subgraphs generated for a third cell type pair. The prediction unit 107 inputs the subgraphs included in each of the subgraph groups 90-1 to 90-3 to the trained responder prediction model 111, predicts a label indicating "responder" or "non-responder" for the input subgraph, and obtains a predicted label 91. The prediction unit 107 obtains multiple predicted labels 91 by predicting a label indicating "responder" or "non-responder" for all of the subgraphs included in the subgraph groups 90-1 to 90-3. Thereafter, the prediction unit 107 aggregates the predicted labels 91 (aggregation 92) to ultimately obtain a prediction result 93 indicating whether the target patient is a responder or a non-responder.

The aggregation 92 of the predicted labels 91 indicating "responder" or "non-responder" is statistical aggregation, and includes, for example, taking a majority vote of the predicted labels 91. In other words, the prediction unit 107 may obtain, as a final prediction result 93, whichever label is indicated the most among the predicted labels 91. Alternatively, the prediction unit 107 may assign "1" to the label indicating "responder" and "0" to the label indicating "non-responder" among the predicted labels 91, and obtain the average value of the total values of the labels as the final prediction result 93. In this case, a value between 0 and 1 is displayed as the prediction result 93 indicating the likelihood of being a responder.

In this way, the information processing apparatus 10 according to the second embodiment obtains spatial information regarding multiple phenotypes of cells that were detected in a medical image and classified, assigns nodes to cells based on the spatial information, connects the nodes with edges, and constructs a graph using features of the nodes and features of the edges. Accordingly, spatial interactions between multiple types of cells in a tumor microenvironment can be analyzed using information on cell positions and interactions. Then, the information processing apparatus 10 trains the responder prediction model 111 using subgraphs extracted from the constructed graph and a true label indicating "responder" or "non-responder". Accordingly, the responder prediction model 111 learns a relationship between a subgraph and a responder or a non-responder. The responder prediction model 111 can learn the relationship between a subgraph and a responder or a non-responder based on cell features and features between cells. Also, the information processing apparatus 10 uses the trained responder prediction model 111 to predict whether a patient is a responder or a non-responder based on subgraphs extracted from a medical image obtained from a target patient, and therefore, high prediction accuracy is expected for such prediction.

In the two embodiments described above, the graph modeling unit 104 extracts multiple subgraphs from the constructed graph as graph-based information, but may extract information other than subgraphs. For example, the graph modeling unit 104 may extract node features and edge features from the constructed graph. In this case, the training data generation unit 105 may train the responder prediction model 111 using the node features, edge features, and a true label.

Also, in the two embodiments described above, the responder prediction model 111 is trained using a true label, which is a label indicating "responder" or "non-responder", but the learning model for performing machine learning may also be trained using other true labels. Specifically, if patients can be classified into a first type of patient and a second type of patient based on medical images, a patient type prediction model (corresponding to the responder prediction model 111 in the embodiments) for machine learning may be trained using a true label indicating the first type of patient or the second type of patient. In this case, the trained learning model can be configured to predict and output information indicating whether a medical image corresponding to a graph corresponding to an input subgraph was obtained from a first type of patient or a second type of patient. Examples of a first type of patient and a second type of patient include a patient with a low survival rate for a given treatment and a patient with a high survival rate for a given treatment (prognosis use case), as well as a cancerous patient and a non-cancerous patient (detection use case).

### Hardware Configuration of Information processing apparatus

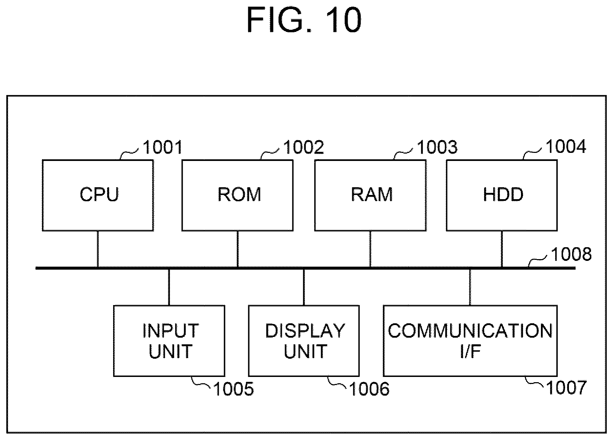

The following describes an example of the hardware configuration of the information processing apparatus 10 configured to be able to implement the above-described embodiments. FIG. 10 is a block diagram showing an example of the hardware configuration of the information processing apparatus 10 according to the embodiments. The information processing apparatus 10 according to the embodiments can be implemented by a single or multiple computers of any type, a single or multiple mobile devices of any type, or any other processing platform. FIG. 10 shows an example in which the information processing apparatus 10 is implemented by a single computer, but the information processing apparatus 10 according to the embodiments may be implemented by a computer system that includes multiple computers. The computers may be communicably connected to each other via a wired or wireless network.

As shown in FIG. 10, the information processing apparatus 10 may include a CPU (Central Processing Unit) 1001, a ROM (Read Only Memory) 1002, a RAM (Random Access Memory) 1003, an HDD (Hard Disk Drive) 1004, an input unit 1005, a display unit 1006, a communication I/F (interface) (communication unit) 1007, and a system bus 1008. The information processing apparatus 10 may also include an external memory. The CPU 1001 performs overall control of operations of the information processing apparatus 10, and controls various components (1002 to 1007) via the system bus 1008, which is a data transmission path.

The ROM 1002 is a non-volatile memory that stores a control program and the like required for the CPU 1001 to execute processing. The program includes instructions (code) for executing the processing according to the above-described embodiments. Note that the program may be stored in a non-volatile memory such as the HDD 1004 or an SSD (Solid State Drive) or an external memory such as a removable storage medium (not shown). The RAM 1003 is a volatile memory and functions as the main memory, work area, and the like of the CPU 1001. In other words, when executing processing, the CPU 1001 loads a necessary program and the like from the ROM 1002 into the RAM 1003, and executes the program and the like to realize various functional operations.

The HDD 1004 stores, for example, various types of data, various types of information, and the like that are required when the CPU 1001 performs processing using the program. The HDD 1004 also stores various types of data, various types of information, and the like that are obtained by the CPU 1001 performing processing using the program and the like. The input unit 1005 is configured by a keyboard and a pointing device such as a mouse. The display unit 1006 is configured by a monitor such as a liquid crystal display (LCD). The display unit 1006 may be configured in combination with the input unit 1005 to function as a GUI (Graphical User Interface).

The communication I/F 1007 is an interface that controls communication performed between the information processing apparatus 10 and an external device. The communication I/F 1007 provides an interface with a network and executes communication with an external device via the network. Various types of data, parameters, and the like are transmitted to and received from an external device via the communication I/F 1007. In the embodiments, the communication I/F 1007 may execute communication via a wired LAN (Local Area Network) that conforms to a communication standard such as Ethernet (registered trademark), or via a dedicated line. However, the network that can be used in the embodiments of the present invention is not limited to this, and may be configured as a wireless network. Examples of the wireless network include a wireless personal area network (PAN) based on, for example, Bluetooth (registered trademark), ZigBee (registered trademark), or UWB (Ultra Wide Band). Other examples include a wireless LAN (Local Area Network) based on Wi-Fi (Wireless Fidelity) (registered trademark), for example, and a wireless MAN (Metropolitan Area Network) based on WiMAX (registered trademark), for example. Further examples include a wireless WAN (Wide Area Network) based on, for example, 4G or 5G. Note that the network may be any network that communicably connects devices to each other, and the communication standard, scale, and configuration of the network are not limited to those described above.

Note that although a specific embodiment has been described above, the embodiment is a mere example and is not intended to limit the scope of the invention. The apparatus and method described in this specification may be implemented in forms aside from the embodiment described above. It is also possible to appropriately make omissions, substitutions, and modifications to the embodiment described above without departing from the scope of the invention. Implementations with such omissions, substitutions, and modifications are included in the scope of the patent claims and their equivalents, and belong to the technical scope of the present invention.

The disclosure includes the following embodiments.
**[1]** An information processing apparatus comprising: a classification unit configured to classify cells in an image of a tumor microenvironment obtained from a patient into a plurality of cell groups; a spatial information extraction unit configured to extract positions, in the image, of the cells included in the plurality of cell groups; a graph creation unit configured to create a graph by assigning nodes to the cells included in the plurality of cell groups based on the extracted positions, connecting the nodes with edges, assigning to each of the nodes a feature quantity indicating a feature of the corresponding cell, and assigning to each of the edges a feature quantity indicating a relationship between two cells corresponding to two nodes connected by the edge; and a graph modeling unit configured to, using a graph neural network (GNN), model spatial relationships between cells in the graph based on the assigned feature quantities of the nodes and the edges.
[2] The information processing apparatus according to [1], wherein the patient is classified into one of a first type of patient and a second type of patient, and the information processing apparatus further comprises: a training unit configured to train a patient type prediction model for machine learning, based on the modeled graph; and a prediction unit configured to, using the trained patient type prediction model, predict whether a target patient is the first type of patient or the second type of patient based on an image of a tumor microenvironment obtained from the target patient.
[3] The information processing apparatus according to [2], wherein the graph modeling unit is further configured to extract a plurality of subgraphs from the modeled graph, and the training unit trains the patient type prediction model to learn a relationship between the plurality of subgraphs and a true label indicating that a patient is the first type of patient or the second type of patient, using the plurality of subgraphs and the true label.
[4] The information processing apparatus according to [3], wherein the graph modeling unit extracts the plurality of subgraphs from the modeled graph on a condition that a subgraph has at least (N+1) nodes (N is an integer greater than or equal to 2) and N hops (N edges continuous with each other).
[5] The information processing apparatus according to any one of [2] to [4], wherein the first type of patient and the second type of patient respectively correspond to a responder to a predetermined treatment and a non-responder to the predetermined treatment.
[6] The information processing apparatus according to any one of [1] to [5], wherein the feature quantity indicating the feature of the corresponding cell includes at least any one of a type of the cell, a size of the cell, a feature of a biomarker expression level in the cell, a relationship with a node corresponding to an adjacent cell, and a center coordinate of the node corresponding to the cell.
[7] The information processing apparatus according to any one of [1] to [6], wherein the feature quantity indicating the relationship between the two cells may include at least any one of a type of connection between the two cells and a distance between the nodes corresponding to the two cells.
[8] The information processing apparatus according to any one or [1] to [7], wherein the plurality of cell groups include a cell group made up of tumor cells and a cell group made up of immune cells.
[9] The information processing apparatus according to any one or [1] to [7], wherein the plurality of cell groups include cell groups classified based on cell phenotype.

## Claims

1. An information processing apparatus comprising:
classification means (101) for classifying cells in an image of a tumor microenvironment obtained from a patient into a plurality of cell groups;
spatial information extraction means (102) for extracting extract positions, in the image, of the cells included in the plurality of cell groups;
graph creation means (103) for creating a graph by assigning nodes to the cells included in the plurality of cell groups based on the extracted positions, connecting the nodes with edges, assigning to each of the nodes a feature quantity indicating a feature of the corresponding cell, and assigning to each of the edges a feature quantity indicating a relationship between two cells corresponding to two nodes connected by the edge; and
graph modeling means (104) for modelling, using a graph neural network (GNN), spatial relationships between cells in the graph based on the assigned feature quantities of the nodes and the edges.

2. The information processing apparatus according to claim 1,
wherein the patient is classified into one of a first type of patient and a second type of patient, and
the information processing apparatus further comprises:
training means (106) for training train a patient type prediction model for machine learning, based on the modeled graph; and
prediction means (107) for predicting, using the trained patient type prediction model, whether a target patient is the first type of patient or the second type of patient based on an image of a tumor microenvironment obtained from the target patient.

3. The information processing apparatus according to claim 2,
wherein the graph modeling means (104) is further configured to extract a plurality of subgraphs from the modeled graph, and
the training means (106) trains the patient type prediction model to learn a relationship between the plurality of subgraphs and a true label indicating that a patient is the first type of patient or the second type of patient, using the plurality of subgraphs and the true label.

4. The information processing apparatus according to claim 3,
wherein the graph modeling means (104) extracts the plurality of subgraphs from the modeled graph on a condition that a subgraph has at least (N+1) nodes (N is an integer greater than or equal to 2) and N hops (N edges continuous with each other).

5. The information processing apparatus according to any one of claims 2 to 4,
wherein the first type of patient and the second type of patient respectively correspond to a responder to a predetermined treatment and a non-responder to the predetermined treatment.

6. The information processing apparatus according to any one of claims 1 to 5,
wherein the feature quantity indicating the feature of the corresponding cell includes at least any one of a type of the cell, a size of the cell, a feature of a biomarker expression level in the cell, a relationship with a node corresponding to an adjacent cell, and a center coordinate of the node corresponding to the cell.

7. The information processing apparatus according to any one of claims 1 to 6,
wherein the feature quantity indicating the relationship between the two cells may include at least any one of a type of connection between the two cells and a distance between the nodes corresponding to the two cells.

8. The information processing apparatus according to any one of claims 1 to 7,
wherein the plurality of cell groups include a cell group made up of tumor cells and a cell group made up of immune cells.

9. The information processing apparatus according to any one of claims 1 to 7,
wherein the plurality of cell groups include cell groups classified based on cell phenotype.

10. An information processing method comprising:
classifying (S21, S61) cells in an image of a tumor microenvironment obtained from a patient into a plurality of cell groups;
extracting (S22, S62) positions, in the image, of the cells included in the plurality of cell groups;
creating (S23, S63) a graph by assigning nodes to the cells included in the plurality of cell groups based on the extracted positions, connecting the nodes with edges, assigning to each of the nodes a feature quantity indicating a feature of the corresponding cell, and assigning to each of the edges a feature quantity indicating a relationship between two cells corresponding to two nodes connected by the edge; and
modeling (S34, S64), using a graph neural network (GNN), spatial relationships between cells in the graph based on the assigned feature quantities of the nodes and the edges.

11. An information processing program for causing a computer to execute information processing, the program causing the computer to execute:
classification processing of classifying cells in an image of a tumor microenvironment obtained from a patient into a plurality of cell groups;
spatial information extraction processing of extracting positions, in the image, of the cells included in the plurality of cell groups;
graph creation processing of creating a graph by assigning nodes to the cells included in the plurality of cell groups based on the extracted positions, connecting the nodes with edges, assigning to each of the nodes a feature quantity indicating a feature of the corresponding cell, and assigning to each of the edges a feature quantity indicating a relationship between two cells corresponding to two nodes connected by the edge; and
modeling processing of modeling, using a graph neural network (GNN), spatial relationships between cells in the graph based on the assigned feature quantities of the nodes and the edges.
